# EUROPÄISCHE PATENTANMELDUNG

(11) **EP 1 640 012 A1**
(43) Veröffentlichungstag der Anmeldung: **29.03.2006**
(21) Anmeldenummer: 04090376.7
(22) Anmeldetag: 24.09.2004
(51) Int. Cl.: A61K 35/14, A61P 37/04

(54) **Pharmazeutisches Mittel enthaltend Blutbestandteile 10 kDa und deren Verwendung zur Prophylaxe und Behandlung von Defekten des Immunsystems**

(71) Anmelder: Salama, Zoser B. nat.rer.Dr., 13465 Berlin (DE)
(72) Erfinder: Zoser, B. Salama Dr., 13465 Berlin (DE)
(74) Vertreter: Hertin, Paul W.

(57) **Zusammenfassung**

Die Erfindung betrifft eine Zusammensetzung aus Proteinen, Peptiden und/oder Peptidkomponenten, ein pharmazeutisches Mittel, umfassend die Zusammensetzung, ein Verfahren zur Herstellung der Zusammensetzung sowie die Verwendung dieser in der Prophylaxe oder Therapie von Personen, Tieren und/oder Patienten mit pathogenen Modifikationen und/oder Defekten der zellulären Immunität, insbesondere Krebs, Sepsis oder allergischen Reaktionen, im Zusammenhang mit einer Zytostatika-, Chemo- und/oder Strahlentherapie.

## Beschreibung

Die Erfindung betrifft eine Zusammensetzung aus Peptid-und/oder Proteinkomponenten der zellulären Bestandteile des Blutes, wobei die Komponenten ein Molekulargewicht von unter 10.000 Da aufweisen, ein pharmazeutisches Mittel umfassend die Zusammensetzung, die Herstellung der Zusammensetzung sowie die Verwendung dieser zur Behandlung von Defekten der zellulären Immunität, insbesondere Krebs, Sepsis, allergische Reaktionen sowie die prophylaktische Verwendung der pharmazeutischen Mittel bei der Behandlung eines Patienten bei Krebs, zum Beispiel mit Zytostatika oder energiereichen Strahlen.

Die Umwelt von Menschen oder Tieren enthält eine große Anzahl von infektiösen Mikroben wie zum Beispiel Viren, Bakterien, Pilzen und Parasiten. Weiterhin können Störungen oder Modifikationen des Stoffwechsels, insbesondere der Zellteilung, dazu führen, dass Individuen sich neben den Umwelteinflüssen auch mit pathologischen Prozessen in ihnen selbst auseinandersetzen müssen, wie zum Beispiel bei der Autoimmunität, bei Krebserkrankungen oder gutartigen Polypenbildungen. Hierbei ist es üblich, dass diese Prozesse - der Auseinandersetzungen mit äußeren und inneren Einflüssen - miteinander wechselwirken wie zum Beispiel bei Entzündungsreaktionen als Ausdruck einer Reaktion auf Pathogene, die aus der Umwelt in Individuen eindringen können und Zellenwucherungen verursachen.

Die genannten Prozesse können pathologische Schädigungen hervorrufen und sie sind im Falle der unkontrollierten Wechselwirkung mit dem Abwehrsystem des Organismus in der Lage, das Individuum - den Wirt - zu töten. Normalerweise ist die Auseinandersetzung mit den Pathogenen zeitlich begrenzt, sie kann jedoch auch bei einer zeitlichen Begrenzung bleibende Schäden des Organismus verursachen. Die zeitliche Begrenzung dieser Reaktionen ist insbesondere dem Immunsystem - als Abwehrsystem des Individuums - zu verdanken.

Immunantworten des Individuums auf Umwelteinflüsse beziehungsweise auf pathogene Veränderungen innerhalb des Individuums lassen sich in zwei große Gruppen einteilen: in humorale und in zelluläre Immunantworten, wobei es häufig nicht möglich ist, einen Krankheitsprozess beziehungsweise einen Genesungsprozess ausschließlich einer dieser beiden Immunantworten zuzuordnen, da sie miteinander wechselwirken und voneinander abhängig sind. Der Ausdruck der zellvermittelten Immunität wurde ursprünglich für diejenigen lokalen Reaktionen gegen Organismen, in der Regel intrazelluläre Pathogene, geprägt, die in erster Linie durch Lymphozyten und Phagozyten und weniger durch Antikörper, die Bestandteil der humoralen Immunität sind, hervorgerufen werden. Mittlerweile wird der Ausdruck der zellvermittelnden Immunität aber für jede Immunantwort gebraucht, in der die Antikörper nicht die zentrale Rolle spielen. Zellvermittelte und antikörpervermittelte Reaktionen können nicht voneinander getrennt betrachtet werden, da beispielsweise auch bei der Entstehung von Antikörpern Zellen beteiligt sind und die Antikörper bei zahlreichen zellvermittelnden Reaktionen als vermittelndes Glied auftreten.
Es ist sogar unwahrscheinlich, dass eine zellvermittelte Reaktion im Sinne der Erfindung initiiert werden kann, wenn Antikörper, die die zellulären Reaktionen auf verschiedenartige Weise beeinflussen können, fehlen beziehungsweise nur in suboptimalen Mengen vorhanden sind.

Die zelluläre Immunantwort ist insbesondere an Makrophagen, B-Zellen, T-Zellen, Lymphozyten, NK-Zellen, Monozyten und zahlreiche andere gebunden.

Die genannten Zellen sind dafür verantwortlich, dass Zytokine, wie zum Beispiel TNF, M-CSF oder GM-CSF freigesetzt werden. Aus dem Zusammenspiel der Zellen, der Zytokine sowie von Umwelteinflüssen und Reaktionen des Individuums selbst, wie zum Beispiel der humoralen Immunität, ergeben sich
- eine mikrobizide und eine tumorizide Aktivität,
- Entzündungsreaktionen und Fieber,
- eine Lymphozytenaktivierung sowie
- eine Gewebereorganisation und Gewebeschädigung.

Hierdurch kann es beispielsweise zum Abtöten von Mikroorganismen, mehrzelligen Parasiten, aber auch zur Abtötung von Tumorzellen und zu Fieberreaktionen kommen.

Aufgrund der vielfältigen Funktionen dieses Bereichs der Immunabwehr gab es zahlreiche Versuche, die Aktivität dieses Systems zu erhöhen. Besonders vorteilhaft erschien es, diesen Bereich der Immunabwehr prophylaktisch in seiner Wirksamkeit zu verbessern, wenn beispielsweise ein Patient einen ernsthaften Unfall hatte oder eine größere Operation durchgeführt werden sollte oder wenn ein Patient mit Zytostatika oder einer Strahlentherapie behandelt wurde. Man ging davon aus, dass die Verbesserung der zellulären Immunantwort Neuinfektionen oder zusätzliche Metastasen verhindern würde beziehungsweise septische und Entzündungsprozesse positiv beeinflussen könnte.

Insbesondere bei der Behandlung von Autoimmunerkrankungen, wie zum Beispiel Psoriasis, atopischen Ekzemen, rheumatoider Arthritis oder der juvenilen Diabetes, ging man von guten Therapiemöglichkeiten aus, sofern die zelluläre Immunantwort in einem Patienten verbessert wird.

Die wachsende Kenntnis über die Rolle des zellulären Immunsystems hat insbesondere bei Krebs zu neuen Therapieansätzen geführt. Mit den so genannten "Krebsimpfungen" wird versucht, das körpereigene zelluläre Abwehrsystem gezielt gegen Tumorzellen auszurichten. Das Ziel der bisherigen zellulären Immuntherapien bei Krebs besteht darin, die Blockade des Immunsystems durch den Tumor aufzuheben und zytotoxische T-Zellen gegen den Tumor zu aktivieren. Diese Prozesse sind jedoch bisher immer noch mit dem Risiko von Autoimmunreaktionen verbunden.

Bekannte Verfahren bestehen darin, zunächst Immunzellen aus dem Blut oder dem Knochenmark zu isolieren und außerhalb des Körpers in einem Reagenzglas zu vermehren, um sie dann wieder in den Patienten zurückzugeben. Hierbei können Zellen des eigenen Körpers verwendet werden (autologe Zellen) oder Zellen eines fremden Spenders (alogene Zellen). Je verschiedener die Zellen von Spender und Empfänger sind, desto höher ist die Wahrscheinlichkeit, dass die übertragenen Zellen die Tumorzellen erkennen können.

Eine weitere Form, um insbesondere die zelluläre Immunantwort zu erhöhen, stellt die Verabreichung von dendritischen Zellen dar. Dendritische Zellen nehmen eine Schlüsselfunktion bei der Aktivierung der Immunantwort ein. Sie präsentieren auffällige Merkmale, die Tumorzellen von anderen Zellen unterscheiden, dem Immunsystem so, dass eine ausgeprägte Reaktion erfolgen kann, die auch zahlenmäßig über einzelne Zellen hinausgeht.

Die dendritischen Zellen werden vom Patienten selbst entnommen und gezielt mit Tumorzellen oder Teilen davon zusammengebracht und dann in den Patienten zurückgegeben. Im Körper sollen die so beladenen dendritischen Zellen dann Tumorzellbruchstücke präsentieren und so eine Immunreaktion gegen den Tumor auslösen.

Auch die Transplantation von Stammzellen aus Knochenmark oder Blut ist eine Möglichkeit der zellulären Immuntherapie. Ursprünglich wurde die Stammzelltransplantation eingeführt, um das durch eine Hochdosis-Behandlung mit Chemotherapie oder Bestrahlung das zerstörte Knochenmark von Leukämiepatienten zu erneuern. Es hat sich aber gezeigt, dass die von einem fremden Spender übertragenen Zellen auch eine direkte Wirkung gegen Krebszellen ausüben, gerade weil sie praktisch nie in allen Gewebemerkmalen mit denen des Empfängers übereinstimmen. Das aus den Spenderzellen gebildete - so genannte neue zelluläre - Immunsystem des Patienten erkennt daher verbleibende Leukämiezellen nun als fremd und bekämpft sie.

Die Aktivierung der zellulären Immunantwort ist aber ein stark regulierter Prozess und erfordert von biochemischer Seite her ein hohes Maß an Energie und ist außerdem mit dem Risiko einer Autoimmunreaktion verbunden. Daher haben derartige klinische Eingriffe das Risiko von Nachteilen und Nebenwirkungen für den Patienten.

Aufgabe der vorliegenden Erfindung ist es daher, eine technische Lehre bereitzustellen, die die genannten Nachteile des Standes der Technik nicht aufweist, und eine einfache, sichere und effiziente Behandlung von Krankheiten ermöglicht, die insbesondere auf Defizite der zellulären Immunantwort zurückzuführen sind, wie zum Beispiel Sepsis, Tumorerkrankungen, Ekzeme, Psoriasis, Neurodermitis und Autoimmunerkrankungen. Aufgabe der vorliegenden Erfindung ist es weiterhin, eine technische Lehre, insbesondere ein pharmazeutisches Mittel bereitzustellen, welches prophylaktisch bei schweren Unfällen, aber auch bei einer Chemotherapeutika- und/oder Strahlenbehandlung mit einem Patienten in Kontakt gebracht werden kann, um dessen Allgemeinstatus - bevorzugt über die Verbesserung der zellulären Immunantwort - zu optimieren. Aufgabe der Erfindung ist es weiterhin, ein Mittel für die prophylaktische Verwendung bereitzustellen und/oder für die Behandlung des Fatique Syndroms als Folge von Schock und/oder physikalischen, emotionalen, nervösen, pathologischen oder radioaktiven Auswirkungen.

Die technische Aufgabe der Erfindung wird gelöst durch ein Verfahren gemäß Anspruch 1, insbesondere zur Herstellung einer Zusammensetzung umfassend vollständige und/oder Bruchteile von Ubiquitin spezifische Protease 32, positiver Kofaktor 2 Glutamin/Q-reiches assoziiertes Protein, Cadherin, Transskriptionsfakor GATA-2, putativer Chromatin-Struktur-Regulator, CUE Domain - enthaltend 1, SUPT6H-Protein, Interleukin 18 Rezeptor 1 Vorläufer, Interleukin 1 Rezeptor-ähnliches Protein und Mucin 4 und/oder Tenascin M, transienter Rezeptor potentiellen Kation-Kanals, Ectonucleotid-Pyrophosphatase/Phospodiesterase, SWI-/SNF-verwandtes matrixassoziiertes Actin-abhängiger Regulator des Chromatins, SWI/SNF Chromatin-modumulierende Komplex-Untereinheit OSA1 B120, OSA1 Kernprotein, ATPase, H+/K+ Wechselpolypeptid, Zink-Finger-Protein 174, Ig-schwere Ketten-V-Region, ADAMTS-3 Vorläufer/Desintegrin-ähnliche und Metalloprotease, Koagulationsfaktor II (Thrombin)Rezeptorähnlich 3, Diacylglycerol Kinase-theta, p250R, SWI/SNF Chromatin-remodulierende Komplex-Untereinheit OSA2, Metallo-phospoesterase, AMP-Deaminase, Alpha-Mannosidase, Cadherin 9, Nik-verwandte Kinase, Alhpa-1B-adrenerger Rezeptor, BRCA1-assoziiertes Protein-1, CD99 Antigen-ähnliche 2 isoform E3-E4, SWHCF-umfassendes Peptid und/oder FAT-ähnliches Cadherin-FATJ, ATP-Bindungskassette, Nucleoporin 153 kDa, ELK3-Protein, Protein ELK-3 ETS-Domäne, ATPase, Kupfer-transportierende Protein-Kinase, Protein Tyrosin-Phosphatase, Integrationsseiten-Familie flügelloser Typ MMTV, MYC Bindungsprotein 2, Cullin 7, gelöste Carrier-Familie 5 (Natriumjodid-Symporter) Mitglied 5, Glutamat-reiche WD Wiederholung enthaltend 1, MAP Kinase interagierende Serin/Threonin Kinase 1, ATP-Bindungskassette, NOV-Plexin-A1 Protein und/oder E3 Ubiquitin Ligase SMURF2, Acyl-CoA Synthetase, Estrogen Sulfotransferase, 2,4-Dienoyl CoA Reductase 1 Vorläufer, 4-Enoyl-CoA Reductase, Claudin 10 Isoform b, DMBT1, extrazellulärer Linker-Domäne enthaltend 1, lymphatischer Endothel-spezifischer Hyaluron-Rezeptor LYVE-1, Rho GTPase aktivierendes Protein 8 Isoform 2, Desintegrin-ähnliches und Metalloprotease (Reprolysintyp) mit Thrombospondin Typ 1 Motiv, Ig-schwere Ketten-V-Region, AS12-Protein, mitochondriales ribosomales Protein S9, 28S ribosomales Protein S9, Protein-Kinase-Substrat MK2S4, NP220, putativer G-Protein-gekoppelter Rezeptor, Dynein-, Axonemal-schweres Polypeptid 5, N-acyl-phosphatidylethanolamin-hydrolysierende Phospholipase D, Leukotrein-B4-Rezeptor, G-Protein-gekoppelter Rezeptor 16, Proprotein Convertase Subtilisin/Kexin Typ-1-Inhibitor CMKRL1, dualspezifische Tyrosin-phosphorylierungs-regulierte Kinase 3, regulatorische Erythroid-Kinase (Langform), DYRK3-Protein, Ig lambda Kette V-VII Region (Mot) - human, Glutathion-Reduktase, mitochondrialer Vorläufer, Collagen alpha 1 (XVI) Kettenvorläuer, 11-beta-Hydroxysteroid Dehydrogenase 1, Insulin-Rezeptor Substrat 2, Vault poly (ADP-ribose) Polymerase (VPARP), Calcium-/Calmodulin-abhängige 3', 5'-zyklische Nucleotide, Zinkfinger-Protein 161, H2.0-ähnliche Homeo-Box-1, H2.0 (Drosophilia)-ähnliche Homeo-Box-1 und/oder Dedicator der Zytokinese 6.

In einer bevorzugten Ausführungsform umfasst die Zusammensetzung unter anderem vollständige und/oder Bruchteile von Interleukin 18 Rezeptor 1 Vorläufer, Interleukin 1 Rezeptor-ähnliches Protein und Mucin 4, transienter Rezeptor potentiellen Kation-Kanals, Ectonucleotid-Pyrophosphatase/Phospodiesterase, SWI-/SNF-verwandtes matrixassoziiertes Actin-abhängiger Regulator des Chromatins, SWI/SNF Chromatin-modumulierende Komplex-Untereinheit OSA1 B120, OSA1 Kernprotein, MYC Bindungsprotein 2, Cullin 7, gelöste Carrier-Familie 5 (Natriumjodid-Symporter) Mitglied 5, Glutamat-reiche WD Wiederholung enthaltend 1, MAP Kinase interagierende Serin/Threonin Kinase 1, ATP-Bindungskassette, DMBT1, extrazellulärer Linker-Domäne enthaltend 1, lymphatischer Endothel-spezifischer Hyaluron-Rezeptor LYVE-1, Rho GTPase aktivierendes Protein 8 Isoform 2, Desintegrin-ähnliches und Metalloprotease (Reprolysintyp) mit Thrombospondin Typ 1 Motiv, AS12-Protein, mitochondriales ribosomales Protein S9, 28S ribosomales Protein S9, Protein-Kinase-Substrat MK2S4, NP220, putativer G-Protein-gekoppelter Rezeptor, Dynein-, Axonemal-schweres Polypeptid 5, N-acyl-phosphatidylethanolamin-hydrolysierende Phospholipase D, Leukotrein-B4-Rezeptor, G-Protein-gekoppelter Rezeptor 16, Proprotein Convertase Subtilisin/Kexin Typ-1-Inhibitor CMKRL1, dualspezifische Tyrosin-phosphorylierungs-regulierte Kinase 3, regulatorische Erythroid-Kinase (Langform), DYRK3-Protein, Ig lambda Kette V-VII Region (Mot) - human.

Es war überraschend, dass die anmeldungsgemäße Zusammensetzung prophylaktisch und therapeutisch sowie zur Unterstützung biologischer Aktivitäten eingesetzt werden kann. Therapeutisch kann die Zusammensetzung, insbesondere in Form einer Kombination von Zusammensetzung und pharmazeutisch akzeptablem Träger als pharmazeutisches Mittel eingesetzt werden, um chronische Infektionen, septische Infektionen, atopische Ekzeme, Neurodermitis, Psoriasis und anderen der oben genannten Immunerkrankungen eingesetzt werden. Eine prophylaktische Indikation der Zusammensetzung ist beispielsweise die Gabe der Zusammensetzung bei einem Patienten, der mit einer Strahlentherapie oder einer Chemotherapie mit Zytostatika behandelt wird, um die Immunsupression, die durch die Art der Therapie initiiert wird, zu verhindern beziehungsweise abzumildern. Aber auch in der präoperativen Phase von Patienten ist eine prophylaktische Gabe der erfindungsgemäßen Zusammensetzung sinnvoll, beispielweise wenn eine Bluttransfusion zu einer zellulären Intoleranz geführt hat, die den Immunstatus des Patienten nachteilig verändert. Selbstverständlich kann der Immunstatus eines Patienten auch nach Unfällen, größerer oder kleinflächiger Verletzungen aber auch nach Traumata nachteilig verändert sein, so dass eine unmittelbare Therapie nicht sofort möglich ist. In diesem Falle kann die erfindungsgemäße Zusammensetzung ebenfalls prophylaktisch eingesetzt werden, um den Zustand des Patienten so zu stabilisieren, dass die Verletzungen oder pathogenen Veränderungen ursächlich therapiert werden können.

Insbesondere unterstützend und therapiebegleitend kann die Zusammensetzung verwendet werden, wenn die Poliferation und Differentation von verschiedenen Zelltypen in verschiedenen Stadien ihrer Reifung optimiert werden soll. Wenn die Freisetzung von CD₄ oder CD₈ und IF-gamma erhöht werden soll beziehungsweise wenn die Aktivität von T-Lymphozyten zu verbessern ist.

Eine weitere Verwendungsmöglichkeit ist die Aktivierung von Thymozytenpopulationen (Th 1) oder die Freisetzung von Zytokinen und Interleukinen. Weiterhin ist es möglich, den Kalzium-Ionen-Transport durch die Zellmembran zu aktivieren oder den oxidativen Metabolismus in Zellen von wichtigen Stoffwechselorganen wie zum Beispiel der Leber oder der Nieren zu verbessern. Aber auch regulatorische Supressionsmechanismen von immunologischen Kaskaden können aktiviert werden.

Selbstverständlich ist es möglich, dass die erfindungsgemäße Zusammensetzung übliche Hilfsstoffe, bevorzugt Träger, Adjuvantien und/oder Vehikel umfassen. Bei den Trägern kann es sich beispielsweise um Füllmittel, Streckmittel, Bindemittel, Feuchthaltemittel, Sprengmittel, Lösungsverzögerer, Resorptionsbeschleuniger, Netzmittel, Adsorptionsmittel und/oder Gleitmittel handeln. In diesem Fall wird die Zusammensetzung insbesondere als Arzneimittel oder pharmazeutisches Mittel bezeichnet.

In einer weiteren bevorzugten Ausführungsform der Erfindung wird das erfindungsgemäße Mittel als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und/oder in dieser Form angewendet. Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen zum Beispiel Polymersubstanzen und Wachse verwendet werden können.

Die Arzneimittel dieser Erfindung können beispielsweise zur oralen Verabreichung in einer beliebigen oral verträglichen Dosierungsform verwendet werden, die Kapseln, Tabletten und wässrige Suspensionen und Lösungen einschließt, aber nicht darauf beschränkt ist. Im Fall von Tabletten zur oralen Verwendung schließen Träger, die häufig verwendet werden, Lactose und Maisstärke ein. Gleitmittel, wie Magnesiumstearat, können typischerweise zugesetzt werden. Zur oralen Verabreichung in Kapselform werden verwendbare Verdünnungsmittel wie Lactose und getrocknete Maisstärke eingesetzt. Wenn wässrige Suspensionen oral verabreicht werden, wird der Wirkstoff mit Emulgier- und Suspendiermitteln kombiniert. Falls gewünscht, können bestimmte Süßmittel und/oder Geschmacksstoffe und/oder Farbmittel zugesetzt werden.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, zum Beispiel tierische und pflanzliche Fette, Wachse, Paraffine, Stärke, Tragant, Cellulosederivate, Polyethylenglycole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, zum Beispiel Milchzucker, Talkum, Kieselsäure, Aluminiumhydroxid, Calciumsilikat und Polyamidpulver oder Gemische dieser Stoffe. Sprays können zusätzlich die üblichen Treibmittel, zum Beispiel Chlorfluorkohlenwasserstoffe, enthalten.

Lösungen und Emulsionen können neben den Wirkstoffen, das heißt der erfindungsgemäßen Zusammensetzung, die üblichen Trägerstoffe wie Lösungsmittel, Lösungsvermittler und Emulgatoren, zum Beispiel Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydofurfurylalkohol, Polyethylenglycole und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben den Wirkstoffen die üblichen Trägerstoffe wie flüssige Verdünnungsmittel, zum Beispiel Wasser, Ethylalkohol, Propylenglykol, Suspendiermittel, zum Beispiel ethoxilierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die Arzneimittel können in Form einer lyophilisierten sterilen injizierbaren Zubereitung, zum Beispiel als sterile injizierbare wässrige oder ölige Suspension, vorliegen. Diese Suspension kann auch mit im Fachgebiet bekannten Verfahren unter Verwendung geeigneter Dispergier- oder Netzmittel (wie zum Beispiel Tween 80) und Suspendiermittel formuliert werden. Die sterile injizierbare Zubereitung kann auch eine sterile injizierbare Lösung oder Suspension in einem ungiftigen parenteral verträglichen Verdünnungs- oder Lösungsmittel, zum Beispiel eine Lösung in 1,3-Butandiol, sein. Zu den verträglichen Vehikeln und Lösungsmitteln, die verwendet werden können, gehören Mannit, Wasser, Ringer-Lösung und isotonische Natriumchloridlösung. Außerdem werden üblicherweise sterile, nichtflüchtige Öle als Lösungsmittel oder Suspendiermedium verwendet. Zu diesem Zweck kann ein beliebiges mildes nichtflüchtiges Öl einschließlich synthetischer Mono- oder Diglyceride verwendet werden. Fettsäuren, wie Ölsäure und ihre Glyceridderivate sind bei der Herstellung von Injektionsmitteln verwendbar, wie es natürliche pharmazeutisch verträgliche Öle, wie Olivenöl oder Rizinusöl, insbesondere in ihren polyoxyethylierten Formen sind. Diese Öllösungen oder Suspensionen können auch einen langkettigen Alkohol oder einen ähnlichen Alkohol als Verdünnungs- oder Dispergiermittel enthalten.

Die genannten Formulierungsformen können auch Färbemittel, Konservierungsstoffe sowie geruchs- und geschmacksverbesserte Zusätze, zum Beispiel Pfefferminzöl und Eukalyptusöl und Süßmittel, zum Beispiel Saccharin, enthalten. Die erfindungsgemäße Zusammensetzung soll in den aufgeführten pharmazeutischen Zubereitungen vorzugsweise in einer Konzentration von etwa 0,01 bis 99,9, vorzugsweise von etwa 0,05 bis 99 Gew.-% der Gesamtmischung vorhanden sein.

Die aufgeführten pharmazeutischen Zubereitungen können außer der Zusammensetzung weitere pharmazeutische Wirkstoffe enthalten; aber neben weiteren pharmazeutischen Wirkstoffen auch Salze, Puffer, Vitamine, Zuckerderivate, insbesondere Saccaride, Enzyme, Pflanzenextrakte und andere. Die Puffer und Zuckerderivate reduzieren mit Vorteil den Schmerz bei subkutaner Applikation und Enzyme wie beispielsweise Hyaluronidase erhöhen die Wirksamkeit. Die Herstellung der oben aufgeführten pharmazeutischen Zubereitungen erfolgt in üblicher Weise nach bekannten Methoden, zum Beispiel durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie der unten genannten Krankheiten angewendet werden. Als geeignete Zubereitung kommen Injektionslösungen, Lösungen und Suspensionen für die orale Therapie, Gele, Aufgussformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können die Arzneimittel in andere Trägermaterialien wie zum Beispiel Kunststoffe - Kunststoffketten zur lokalen Therapie -, Kollagen oder Knochenzement eingearbeitet werden.

In einer weiteren bevorzugten Ausführungsform der Erfindung ist die Zusammensetzung in einer Konzentration von 0,1 bis 99,5, bevorzugt von 0,5 bis 95, besonders bevorzugt von 20 bis 80 Gew.-% in einer pharmazeutischen Zubereitung eingebracht. Das heißt, die Zusammensetzung ist in den oben aufgeführten pharmazeutischen Zubereitungen, zum Beispiel Tabletten, Pillen, Granulaten und anderen, vorzugsweise in einer Konzentration von 0,1 bis 99,5 Gew.-% der Gesamtmischung vorhanden. Die Wirkstoffmenge, das heißt die Menge einer erfindungsgemäßen Zusammensetzung, die mit den Trägermaterialien kombiniert wird, um eine einzige Dosierungsform zu erzeugen, wird von dem Fachmann in Abhängigkeit von dem zu behandelnden Patienten und der besonderen Verabreichungsart variieren können. Nach Besserung des Zustandes des Patienten kann der Anteil der wirksamen Verbindung in der Zubereitung so geändert werden, dass eine Erhaltungsdosis vorliegt, die die Krankheit zum Stillstand bringt. Anschließend kann die Dosis oder Frequenz der Verabreichung oder beides als Funktion der Symptome auf eine Höhe verringert werden, bei der der verbesserte Zustand beibehalten wird. Wenn die Symptome auf das gewünschte Niveau gelindert worden sind, sollte die Behandlung aufhören. Patienten können jedoch eine Behandlung mit Unterbrechung auf Langzeitbasis nach beliebigem Wiederauftreten von Erkrankungssymptomen benötigen. Demgemäß ist der Anteil der Zusammensetzung, das heißt ihre Konzentration, in der Gesamtmischung der pharmazeutischen Zubereitung ebenso wie ihre Zusammensetzung oder Kombination variabel und kann vom Fachmann aufgrund seines Fachwissens modifiziert und angepasst werden.

Dem Fachmann ist bekannt, dass die erfindungsgemäße Zusammensetzung mit einem Organismus, bevorzugt einem Menschen oder einem Tier, auf verschiedenen Wegen in Kontakt gebracht werden können. Weiterhin ist dem Fachmann bekannt, dass insbesondere die pharmazeutischen Mittel in verschiedenen Dosierungen appliziert werden können. Die Applikation sollte hierbei so erfolgen, dass die Erkrankung möglichst effektiv bekämpft wird bzw. der Ausbruch einer Krankheit in einer prophylaktischen Gabe verhindert wird. Die Konzentration und die Art der Applikation können vom Fachmann durch Routineversuche eruiert werden. Bevorzugte Applikationen der erfindungsgemäßen Verbindungen sind die orale Applikation in Form von Pulver, Tabletten, Saft, Tropfen, Kapseln oder ähnlichem, die rektale Applikation in Form von Zäpfchen, Lösungen und ähnlichem, parenteral in Form von Injektionen, Infusionen und Lösungen sowie lokal in Form von Salben, Pflastern, Umschlägen, Spülungen und ähnlichem. Bevorzugt erfolgt das In-Kontakt-Bringen der erfindungsgemäßen Zusammensetzung prophylaktisch oder therapeutisch.

Die Eignung der gewählten Applikationsformen wie auch der Dosis, des Applikationsschemas, der Adjuvantswahl und dergleichen kann beispielsweise durch Entnahme von Serum-Alliquoten aus dem Patienten, das hießt dem Mensch oder dem Tier, und dem Testen auf das Vorhandensein von Krankheitsindikatoren im Verlauf des Behandlungsprotokolls bestimmt werden. Alternativ und begleitend hierzu kann der Zustand der Nieren, der Leber o.ä., aber auch die Menge von T-Zellen oder anderen Zellen des Immunsystems, auf herkömmliche Weise begleitend bestimmt werden, um einen Gesamtüberblick über die immunologische Konstitution des Patienten und insbesondere die Konstitution von stoffwechselwichtigen Organen, zu erhalten. Zusätzlich kann der klinische Zustand des Patienten auf die gewünschte Wirkung hin beobachtet werden. Wenn eine unzureichende therapeutische Effektivität erzielt wird, kann der Patient mit erfindungsgemäßen Mitteln ggf. mit anderen bekannten Medikamenten modifiziert weiterbehandelt werden, von denen eine Verbesserung der Gesamtkonstitution erwartet werden kann. Selbstverständlich ist es auch möglich, die Träger oder Vehikeln des pharmazeutischen Mittels zu modifizieren oder den Verabreichungsweg zu variieren.

Neben der oralen Aufnahme kann es dann zum Beispiel vorgesehen sein, dass Injektionen beispielsweise intramuskulär oder subkutan oder in die Blutgefäße ein weiterer bevorzugter Weg für die therapeutische Verabreichung der erfindungsgemäßen Zusammensetzung sind. Zeitgleich kann auch die Zufuhr über Katheter oder chirurgische Schläuche angewendet werden; beispielsweise über Katheter, die direkt zu bestimmten Organen wie den Nieren, der Leber, der Milz, dem Darm, der Lunge etc. führen.

Die erfindungsgemäße Zusammensetzung kann in einer bevorzugten Ausführungsform in einer Gesamtmenge von bevorzugt 0,05 bis 500 mg/kg Körpergewicht je 24 Stunden eingesetzt werden, bevorzugt von 5 bis 100 mg/kg Körpergewicht. Hierbei handelt es sich vorteilhafterweise um eine therapeutische Menge, die verwendet wird, um die Symptome einer Störung oder responsiven, pathologisch physiologischen Kondition zu verhindern oder zu verbessern.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,05 bis 500 mg/kg Körpergewicht kann einmalig oder mehrfach angewendet werden, um die gewünschten Ergebnisse zu erhalten. Typischerweise werden insbesondere pharmazeutischen Mittel zur etwa 1- bis 10-maligen Verabreichung pro Tag oder alternativ oder zusätzlich als kontinuierliche Infusion verwendet. Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Targetsdosis auf 2 bis 5 Applikationen zu verteilen, wobei bei jeder Applikation zum Beispiel 1 bis 2 Tabletten mit einem Wirkstoffgehalt von 0,05 bis 500 mg/kg Körpergewicht verabreicht werden. Selbstverständlich ist es möglich, den Wirkstoffgehalt auch höher zu wählen, beispielsweise bis zu einer Konzentration bis 5000 mg/kg. Die Tabletten können auch retardiert sein, wodurch sich die Anzahl der Applikationen pro Tag auf 1 bis 3 vermindert. Der Wirkstoffgehalt der retardierten Tabletten kann 3 bis 3000 mg betragen. Wenn der Wirkstoff - wie ausgeführt - durch eine Injektion verabreicht wird, ist es bevorzugt, 1- bis 10-mal pro Tag beziehungsweise durch Dauerinfusion den Wirt mit der erfindungsgemäßen Zusammensetzung in Kontakt zu bringen, wobei Mengen von 1 bis 4000 mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens erfolgen.

In einer weiteren besonders bevorzugten Ausführungsform der Erfindung wird das pharmazeutische Mittel in einer Einzelgabe von 1 bis 100, insbesondere von 2 bis 50 mg/kg Körpergewicht eingesetzt. Wie auch die Gesamtmenge pro Tag (siehe oben) kann auch die Menge der Einzelgabe pro Applikation von dem Fachmann aufgrund seines Fachwissens variiert werden. Die erfindungsgemäß verwendeten Verbindungen können in den genannten Einzelkonzentrationen und Zubereitungen zusammen mit dem Futter bzw. mit Futterzubereitungen oder mit dem Trinkwasser auch in der Veterinärmedizin gegeben werden. Eine Einzeldosis enthält vorzugsweise die Menge Wirkstoff, die bei einer Applikation verabreicht wird, und die gewöhnlich einer ganzen, einer halben Tagesdosis oder einem Drittel oder einem Viertel einer Tagesdosis entspricht. Die Dosierungseinheiten können demgemäß bevorzugt 1, 2, 3 oder 4 oder mehrere Einzeldosen oder 0,5, 0,3 oder 0,25 einer Einzeldosis enthalten. Bevorzugt wird die Tagesdosis der erfindungsgemäßen Verbindungen auf 2 bis 10 Applikationen verteilt, bevorzugt auf 2 bis 7, besonders bevorzugt auf 3 bis 5 Applikationen. Selbstverständlich ist auch eine Dauerinfusion der erfindungsgemäßen Mittel möglich.

In einer besonders bevorzugten Ausführungsform der Erfindung werden bei jeder oralen Applikation der erfindungsgemäßen Verbindungen 1 bis 2 Tabletten gegeben. Die erfindungsgemäßen Tabletten können mit dem Fachmann bekannten Überzügen und Hüllen versehen sein und auch so zusammengesetzt werden, dass sie den oder die Wirkstoffe nur bei bevorzugten, in einem bestimmten Teil des Wirts freigeben.

Bevorzugt ist es in einer weiteren Ausführungsform der Erfindung, dass die einzelnen Bestandteile der Zusammensetzung gegebenenfalls miteinander assoziiert oder mit einem Träger verbunden in Liposomen eingeschlossen sind, wobei der Einschluss in Liposomen im Sinne der Erfindung nicht zwingend bedeuten muss, dass die Zusammensetzung im Inneren der Liposomen vorliegt. Ein Einschluss im Sinne der Erfindung kann auch bedeuten, dass die Zusammensetzung mit der Membran der Liposomen assoziiert ist, beispielsweise so, dass diese auf der äußeren Membran verankert sind. Eine solche Darstellung der erfindungsgemäßen Zusammensetzung in oder auf den Liposomen ist vorteilhaft, wenn der Fachmann die Liposomen so auswählt, dass sie eine immunstimulierende Wirkung haben. Dem Fachmann sind aus der DE 198 51 282 verschiedene Möglichkeiten bekannt, die immunstimulierende Wirkung von Liposomen zu modifizieren. Bei den Lipiden kann es sich um einfache Lipide handeln, wie beispielsweise Ester und Amide oder um komplexe Lipide wie zum Beispiel um Glycolipide wie Cerebroside oder Ganglioside, um Sphingolipide oder um Phospholipide.

Die Erfindung betrifft auch ein Verfahren zur Herstellung einer Zusammensetzung, die für die genannten prophylaktischen und therapeutischen Indikationen eingesetzt werden kann sowie für therapiebegleitende Verbesserungen der biologischen Effizienz, insbesondere des zellulären Immunsystems.

Das erfindungsgemäße Verfahren besteht darin, Blut-, Plasma-oder Serumbestandteile, die weitestgehend keine immunologischen Toleranzprobleme hervorrufen, zu sammeln und zu homogenisieren. Das Homogenisieren kann beispielsweise mechanisch erfolgen und/oder durch Gefrier-Tauzyklen oder durch andere dem Fachmann bekannte Homogenisierungsmethoden. Weiterhin kann es vorteilhaft sein, die Ausgangskomponenten zur Herstellung der Zusammensetzung oder die Zusammensetzung selbst erst einzufrieren und dann zu schneiden, beispielsweise mit einem Mikrotom.

Homogenisierungen sind im Sinne der Erfindung alle Verfahren, die eine Zelllyse induzieren oder unterstützen können. Durch die Homogenisierung wird das innige Vermengen von an sich nicht oder schwer mischbaren Komponenten eines Systems über das gesamte Volumen ermöglicht, so dass das gewonnene Material weitestgehend unabhängig von der Anzahl der Bestandteile im Wesentlichen nur in wenigen Phasen, insbesondere in einer einzigen, auftritt. Die Homogenisierung bewirkt eine Verkleinerung der Teilchengröße der dispersen Phase, eine Desagglomerisation von Teilchenaggregaten und führt zu Dispersionen mit erhöhter Sedimentationsstabilität. Die Homogenisierung kann mit dynamischen Apparaten aber auch mit statischen Apparaten, das heißt in Mischern ohne bewegliche Teile, erfolgen. Bevorzugtes Ausgangsmaterial sind Blutzellen, bevorzugt weiße Blutzellen. Die Blutzellen können beispielsweise als Buffy-Coat-Konserve, welche reich an Thrombo- und Erytrozyten sind, präpariert werden, wobei dem Fachmann Standardverfahren zur Herstellung dieser Konserven bekannt sind. Selbstverständlich ist es auch möglich, die homogenisierte Probe als Leukozytenkonzentrat zu gewinnen.

Es kann außerdem vorteilhaft sein, das Startmaterial beispielsweise rote und/oder weiße Blutkörperchen, zunächst als Buffy-Coat-Konserve zu präparieren, um die biologisch aktiven Komponenten zum Beispiel durch einen Gefrier-Tau-Zyklus zu homogenisieren. Das heißt die genaue Abfolge der einzelnen Verfahrensschritte im Sinne der Erfindung ist stauschbar. Durch Dialyse, Zentrifugation und/oder Filtration werden von dem - durch Gefrier-Tau-Zyklus oder andere Homogenisationsverfahren - gewonnenen Produkt im Wesentlichen alle Bestandteile abgetrennt, die größer sind als 10.000 Dalton.

Es kann vorteilhaft sein, dass so gewonnene Dialyse-, Zentrifugations- oder Filtrationsprodukt aufzukonzentrieren. In einer vorteilhaften Ausführungsform der Erfindung werden zunächst zelluläre Blutbestandteile, wie zum Beispiel Leukozyten, homogenisiert und anschließend dialysiert. Danach erfolgt eine Lyophilization. Anschließend kann mit Vorteil das Herstellen einer Lösung erfolgen, die zunächst vorfiltriert, dann ultrafiltriert und anschließend steril filtriert wird.

Das gewonnene Filtrat kann beispielsweise in einem Wasserbad pasteurisiert werden. Nach diesem Prozess kann das pasteurisierte Material sterilisiert und wiederum lyophilisiert werden. Selbstverständlich können auch andere Sterilisationsmethoden verwendet werden wie beispielsweise die Behandlung mit energiereicher Strahlung zum Beispiel UV-oder Röntgenstrahlung. Jeder dieser genannten einzelnen Schritte kann durch Qualitätskontrollen begleitet werden. Diese sind beispielsweise dadurch möglich, dass Aliquots aus der Probe entnommen werden, die auf das Vorhandensein von Mikroorganismen, Viren oder anderen nicht erwünschten Bestandteilen hin untersucht werden.

Bevorzugt erfolgt die Präparation des Blutzellenkonzentrates, insbesondere des Leukozytenkonzentrates durch Gefrier-Tauzyklen beziehungsweise durch Ultraschallbehandlung der Zellen beziehungsweise als Kombination dieser beiden Verfahren.

Insbesondere der Gefrier-Tau-Zyklus ermöglicht es, stabile und reproduzierbare Bruchstücke aus den genannten Proteinen und Peptiden der zellulären Bestandteile des Blutes zu gewinnen. Die erfindungsgemäße Zusammensetzung stellt die lyophilisierbaren, sterilisierbaren, filtrierbaren und dialysierbaren Homogenate aus Blutzellen, insbesondere weißen Blutzellen dar, die mehrfach einen Gefrier-Tau-Zyklus durchlaufen haben und bei Temperaturen um 100 °C sterilisiert wurden. Der Gefrier-Tau-Zyklus kann hierbei so ausgestaltet sein, dass das gefrorene Material geschnitten wird, z. B. mit einem Mikrotom, um es dann aufzutauen und gegebenenfalls wieder einzufrieren. Bevorzugt ist der Gefrier-Tau-Zyklus also ein Gefrier-Schneiden-Tau-Zyklus. Die Verfahrensbedingungen wurden so gewählt, dass nur die stabilen Bruchstücke der Peptide und Proteine in der gewonnenen Zusammensetzung vorhanden sind.

Die Dialyse des Homogenisates erfolgt so, dass niedermolekulare Teilchen von Kolloiden oder Makromolekülen mittels Diffusion aus dem Homogenat in das bevorzugt laufend reine Lösungsmittel durch semipermeable Membran dringen, wobei die großen Moleküle zurückgehalten werden. Die Geschwindigkeit der Dialyse lässt sich durch Temperaturerhöhung oder das Anlegen einer elektrischen Spannung, zum Beispiel bei der Elektrodialyse, vergrößern. Die Dialyse kann selbstverständlich auch mit Dialysesäulen erfolgen, wobei Moleküle, mit einem Molekulargewicht von über 10 kDa abgetrennt werden.

Die Gefriertrocknung dient insbesondere der Aufkonzentrierung des Dialysates. Die Gefriertrocknung im Sinne der Erfindung ist eine Bezeichnung für das Trocknen eines tiefgefrorenen Materials im Hochvakuum durch das Ausfrieren des Lösungsmittels, das dann im gefrorenen Zustand durch die Sublimationstrocknung verdampft. Die Gefriertrocknung im Sinne der Erfindung kann auch als Dehydratisierung insbesondere unter Einsatz von Lösungen erfolgen; bevorzugt unter Zusatz von Lösungen wie zum Beispiel Serum, Milch, Kohlenhydraten, Aminosäuren, Enzymen, Pufferlösungen, Salzen und/oder Vitaminen. Um das lyophilisierte Material für die Anwendung wieder in Lösung zu bringen, ist ein Auflösen beispielsweise in Aqua dest oder in anderen Lösungsmitteln möglich.

Es empfiehlt sich, nach der Präparation dieser Lösung das Ultraviolettspektrum des Materials zu bestimmen, insbesondere im Bereich von 200 nm und 400 nm. Sofern das Material frei von unerwünschten Bestandteilen beziehungsweise weitestgehend frei von solchen Bestandteilen ist, ist eine Präfiltration zum Beispiel durch eine Millipore-Membran vorteilhaft. Bei diesem Verfahren werden Feststoffteilchen aus Flüssigkeiten abgetrennt. Hierbei wird ein poröses Medium, wie beispielsweise die Millipore-Membran RA (1,2 µm) mit einem Präfilter AP15, von der kontinuierlichen Phase der Flüssigkeit durchströmt, wobei gleichzeitig die dispergierte Phase an der Oberfläche des porösen Mittels oder in seinem Inneren zurückgehalten werden.

Mittels einer Ultrafiltration kann Material mit einem Grenzwert von 10 kDa abgetrennt werden. Vorteilhafterweise kann das gewonnene Material durch einen weiteren Filtrationsschritt sterilisiert werden, beispielsweise mit Hilfe des Sterilisationsfilters Millipore GS (0,22 µm). Die Ultrafiltration kann durch Membran-Mikrofiltration aber auch durch Umkehrosmose erfolgen. Für die Ultrafiltration kommen vorwiegend asymmetrisch strukturierte, poröse Membranen verschiedener organischer und anorganischer Materialien zum Einsatz, wie zum Beispiel Polysulphon oder Keramik in Form von Schläuchen, Kapillaren, Hohlfasern und Flächenmembranen.

Es ist vorteilhaft, das so gewonnene sterilisierte Material durch Hitzeinaktivierung zu pasteurisieren. Die Pasteurisierung kann beispielsweise in einem Wasserbad bei einer Temperatur von 60 °C für mehrere Stunden erfolgen. Selbstverständlich kann die Pasteurisierung aber bei jeder Temperatur unter 100 °C in bestimmten Fällen aber auch über 100 °C für eine beliebige Dauer durchgeführt werden. Das heißt, auch eine Sterilisation von über 100 °C ist eine Pasteurisierung im Sinne der Erfindung.

Die Erfindung betrifft auch die Verwendung der erfindungsgemäßen Zusammensetzung und/oder des erfindungsgemäßen pharmazeutischen Mittels zur Behandlung von Krankheiten, die mit einer Defizienz der zellulären Immunität verbunden sind, zum Beispiel bei dem Defekt nach ICD10-Code: D.84.4. Hierbei kann es sich um Sepsiserkrankungen handeln, um Entzündungsreaktionen und Fieber, um Autoimmun-Erkrankungen und Erkrankungen der Störung der Zellteilung wie zum Beispiel Krebs.

Entzündungen im Sinne der Erfindung sind die vom Bindegewebe und den Blutgefäßen getragene Reaktion des Organismus auf einen äußeren oder innerlich ausgelösten Entzündungsreiz mit dem Zweck, diesen zu beseitigen oder zu inaktivieren und die reizbedingte Gewebsschädigung zu reparieren. Auslösend wirken mechanische Reize (Fremdkörper, Druck, Verletzung) und andere physikalische Faktoren (ionisierende Strahlen, UV-Licht, Wärme, Kälte), chemische Stoffe (Laugen, Säuren, Schwermetalle, bakterielle Toxine, Allergene und Immunkomplexe) sowie Erreger(Mikroorganismen, Würmer, Insekten) bzw. krankhafte Stoffwechselprodukte, entgleiste Enzyme, bösartige Tumoren. Das Geschehen beginnt mit einer kurzen Arteriolenverengung (durch Adrenalinwirkung) mit Mangeldurchblutung und Gewebsalteration, gefolgt von der Entwicklung der klassischen örtlichen Entzündungszeichen (Kardinalsymptome; nach GALEN und CELSUS), das heißt von Rötung (= Rubor; Gefäßerweiterung durch Histamin), Wärme (= Calor; durch örtliche Stoffwechselsteigerung), Schwellung (= Tumor; durch Austritt eiweißreicher Flüssigkeit aus den - unter anderem durch Histamin - veränderten Gefäßwänden, unterstützt durch die verlangsamte Blutzirkulation im Sinne der Prästase bis Stase), Schmerz (= Dolor; als Folge der erhöhten Gewebsspannung und schmerzauslösender Entzündungsprodukte, zum Beispiel Bradykinin) und Funktionsstörung (= Functio laesa). Der Vorgang wird ergänzt durch Störung des Elektrolythaushaltes (Transmineralisation), Einwanderung neutrophiler Granulozyten und Monozyten durch die Gefäßwände (s.a. Leukotaxis), letzteres mit dem Zweck, den Entzündungsreiz und geschädigte bis neukrotische Zellen zu beseitigen (Phagozytose); ferner wandern Lymphozyten-Effektorzellen ein, die zur Bildung spezifischer Antikörper gegen den Entzündungsreiz führen (Immunreaktion), sowie Eosinophile (in der Heilungsphase bzw. - sehr frühzeitig - bei allergischhyperergischem Geschehen). Durch die bei der Reaktion erfolgende Aktivierung des Komplementsystems werden Bruchstücke (C3a und C5a) dieses Systems frei, die - wie das Histamin und Bradykinin - als Mediatoren der Entzündung wirken, und zwar im Sinne der Anregung der Chemotaxis der zitierten Blutzellen; ferner wird die Blutgerinnung aktiviert. In der Folge tritt eine Schädigung (Dystrophie und Koagulationsnekrose) des zugeordneten Organparenchyms ein. Der Gesamtorganismus reagiert je nach Intensität und Art der Entzündung mit Fieber, Stress (s.a. Adaptationssyndrom), Leukozytose und Veränderungen in der Zusammensetzung der Plasmaproteine (Akute-Phase-Reaktion), die zu einer beschleunigten Blutkörperchensenkungsreaktion führen. Bevorzugte Entzündungen im Sinne der Erfindung sind die eitrige, die exudative, die fibrinöse, die gangräneszierende, die granulomatöse, die hämorrhagische, die katarrhalische, die nekrotisierende, die proliferative oder produktive, die pseudomembranöse, die seröse, die spezifische und/oder die ulzeröse Entzündungen.

Autoimmunerkrankungen im Sinne der Erfindung sind Krankheiten, die ganz oder teilweise auf die Bildung von Autoantikörpern und deren schädigende Einwirkung auf den Gesamtorganismus bzw. Organsysteme, das heißt auf Autoaggression zurückzuführen sind. Eine Klassifikation ist als organspezifische, intermediäre und/oder systemische Autoimmunerkrankung möglich. Bevorzugte organspezifische Autoimmunerkrankungen sind HASHIMOTO Thyreoiditis, primäres Myxödem, Thyreotoxikose (BASEDOW Krankheit), perniziöse Anämie, ADDISON Krankheit, Myasthenia gravis und/oder juveniler Diabetes mellitus. Bevorzugte intermediäre Autoimmunkrankheiten sind GOODPASTURE Syndrom, autoimmune hämolytische Anämie, autoimmune Leukopenie, idiopathische Thrombozytopenie, Pemphigus vulgaris, sympathische Ophthalmie, primäre biliäre Zirrhose, Autoimmunhepatitis, Colitis ulcerosa und/oder SJÖGREN Syndrom. Bevorzugte systemische Autoimmunkrankheiten sind rheumatoide Arthritis, rheumatisches Fieber, systemischer Lupus erythematodes, Dermatomyositis/Polymyositis, progressive systemische Sklerose, WEGENER Granulomatose, Panarteriitis nodosa und/oder Hypersensitivitätsangiitis. Typische Autoimmunkrankheiten sind Thyreotoxikose, Schilddrüsen-bedingtes Myxödem, HASHIMOTO Thyreoiditis, generalisierte Endokrinopathie, perniziöse Anämie, chronische Gastritis Typ A, Krankheiten einzelner oder aller korpuskulären Elemente des Blutes (zum Beiapiel autoimmunhämolytische Anämie, idiopath. Thrombozytopenie bzw. -pathie; idiopath. Leukopenie bzw. Agranulozytose), Pemphigus vulgaris und Pemphigoid, sympathische Ophthalmie und manche Uveitis-Formen, primär biliäre Leberzirrhose und chronisch aggressive Autoimmunhepatitis, Diabetes mellitus Typ I, CROHN Krankheit und Colitis ulcerosa, SJÖGREN Syndrom, ADDISON Krankheit, Lupus erythematodes disseminatus und als diskoide Form dieser Krankheit, als Dermatomyositis und Sklerodermie, rheumatoide Arthritis (= primär-chronische Polyarthritis), Antiglomerulusbasalmembran-Nephritis. Grundlage sind eine aggressive Immunreaktion infolge Zusammenbruchs der Immuntoleranz gegenüber Selbst-Derterminanten und eine Abnahme der Aktivität der T-Suppressorzellen (mit Lymphozytenmarker T 8) bzw. ein Übergewicht der T-Helferzellen (mit Lymphozytenmarker T 4) über die Suppressorzellen; ferner ist die Bildung von Autoantigenen möglich, zum Beispiel durch Verbindung von Wirtsproteinen mit Haptenen (zum Beispiel Arzneimittel), durch ontogenetisches Gewebe, das sich erst nach Entwicklung der Selbsttoleranz entwickelt und für durch Änderungen der Konformation der Proteine demaskierte Proteinkomponenten im Zusammenhang zum Beispiel mit Infektion durch Viren oder Bakterien; ferner für im Zusammenhang mit Neoplasien entstandene neue Proteine.

Sepsiserkrankungen sind im Sinne der Erfindung Erkrankungen infolge dauernden oder periodischen Eindringens von pathogenen Bakterien und/oder deren Giften aus einem Krankheitsherd und deren Ausbreitung auf dem Lymph-Blut-Weg zur allgemein beziehungsweise lokalen Infektion.

Sepsis im Sinne der Erfindung sind bevorzugt Wundsepsis (Phlegmone, Thrombophlebitis, Lymphangitis), Puerperalsepsis (bei Puerperalfieber), otogene Sepsis (bei Otitis media), tonsillogene Sepsis (bei Angina, Peritonsillitis), cholangitische Sepsis (bei eitriger Cholezystzitis, Cholangitis), pylephlebitische Sepsis (bei Pylephlebitis) Nabelsepsis (bei Omphalitis etc.), Urosepsis sowie bei Zahngranulom. Die Sepsis im Sinne der Erfindung kann akut bis hochakut (foudroyant), subakut (z. B. als Endocarditits lenta) oder chronisch erfolgen, aber selbstverständlich auch als Neugeborenensepsis.

Sepsis im Sinne der Erfindung sind also alle pathogenen Veränderungen eines Patienten, die mit intermitterierendem Fieber und Schüttelfrost verbunden sein können sowie mit Milztumor, mit toxischen Reaktionen beziehungsweise Schäden des Knochenmarks beziehungsweise des Blutes (polynukleäre Leukozytose, Anämie, Hämolyse, Thrombozytopenie) oder aber mit pathogenen Reaktionen am Herzen und der Gefäßnerven (Tachykardie, Zentralisation des Kreislaufs, Ödeme, Oligurie; evtl. Schock) beziehungsweise des Verdauungstraktes (trockene, belegte Zunge, Durchfälle) oder aber mit Septikopyämie (Pyämie mit Bildung septischer Infarkte und metastatischer Abszess).

Bevorzugte Erkrankungen, die mit einer Defizien des zellulären Immunsystems verbunden sind, sind im Sinne der Erfindung weiterhin: AIDS, Akne, Albuminurie (Proteinurie), Alkoholentzugssyndrom, Allergien, Alopezie (Haarausfall), ALS (Amyotrophe Lateralsklerose), Alzheimer Erkrankung, AMD (Altersbedingte Makuladegeneration), Anämie, Angststörungen, Anthrax (Milzbrand), Aortensklerose, arterielle Verschlusskrankheit, Arterienverkalkung, Arterienverschluss, Arteriitis temporalis, Arteriosklerose, Arteriovenöse Fisteln, Arthritis, Arthrose, Asthma, Ateminsuffizienz, Autoimmunerkrankung, AV-Block, Azidose, Bandscheibenvorfall, Bauchfellentzündung, Bauchspeicheldrüsenkrebs, Becker Muskeldystrophie, Benigne Prostata Hyperplasie (BPH), Blasenkarzinom, Bluterkrankheit (Hämophilie), Bronchialkarzinom, Brustkrebs, BSE, Budd-Chiari-Syndrom, Bulimia nervosa, Bursitis (Schleimbeutelentzündung), Byler-Syndrom, Bypass, Chlamydien-Infektion, Chronische Schmerzen, Cirrhose, Commotio cerebri (Gehirnerschütterung), Creutzfeld-Jakob-Erkrankung, Darmkarzinom, Darmkrebs, Darmtuberkulose, Depression, Diabetes insipidus, Diabetes mellitus, Diabetes mellitus juvenilis, Diabetische Retinopathie, Duchenne-Muskeldystrophie, Duodenalkarzinom, Dystrophia musculorum progressiva, Dystrophie, Ebola, Ekzem, Erektile Dysfunktion, Fettsucht, Fibrose, Gebärmutterhalskrebs, Gebärmutterkrebs, Gehirnblutung, Gehirnentzündung, Haarausfall, Halbseitenlähmung, Hämolytische Anämie, Hämophilie, Haustierallergie (Tierhaarallergie), Hautkrebs, Herpes zoster, Herzinfarkt, Herzinsuffizienz, Herzklappenentzündung, Hirnmetastase, Hirnschlag, Hirntumor, Hodenkrebs, Ischämie, Kahler-Krankheit (Plasmozytom), Kinderlähmung (Poliomyelitis), Knochenschwund, Kontaktekzem, Lähmung, Leberzirrhose, Leukämie, Lungenfibrose, Lungenkrebs, Lungenödem, Lymphdrüsenkrebs (Morbus Hodgkin), Lymphogranulomatose, Lymphom, Lyssa, Magenkarzinom, Mammakarzinom, Meningitis, Milzbrand, Mukoviszidose (zystrische Fibrose), Multiple Sklerose (MS), Myokardinfarkt, Neurodermitis, Neurofibromatose, Neuronale Tumoren, Nierenkrebs (Nierenzellkarzinom), Osteoporose, Pankreaskarzinom, Pneumonie, Polyneuropathien, Potenzstörungen, Progressive Systemische Sklerose (PSS), Prostatakrebs, Quaddelauschlag (Urtikaria), Querschnittssyndrom, traumatisches, Rektumkarzinom, Rippenfellentzündung, Schädel-Hirn-Trauma, Scheidenkrebs (Vaginalkarzinom), Sinusitis, Speiseröhrenkrebs, Tremor, Tuberkulose, Tumorschmerz, Vaginalkarzinom, Verbrennung - Verbrühung, Vergiftungen, Virale Meningitis, Wechseljahre, Weichteilsarkom, Weichteiltumor, Zerebrale Durchblutungsstörungen und/oder ZNS-Tumore.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppression-bezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchiai-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-IIassoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholangiom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoli-Zell-Tumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nichtchromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (zum Beispiel Ewing-Sarkom, experimentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungenmetastasen, Lebermetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzellkarzinome und/oder Lebermetastasen.

Die Erfindung betrifft auch die Verwendung der erfindungsgemäßen Zusammensetzung in Verfahren zur Prophylaxe und/oder Therapie von Personen, Tieren und/oder Patienten mit pathogenen Modifikationen und/oder Defekten der zellulären Immunität, insbesondere Krebs, Sepsis, allergischen Reaktionen im Zusammenhang mit einer Zytostatika-, Chemo-und/oder Strahlentherapie und/oder als Prophylaxe und/oder Therapie im Zusammenhang mit Unfällen mit atomaren, biologischen, chemischen und/oder radioaktiven Stoffen und/oder Materialien.

Die Erfindung betrifft auch einen Kit und dessen Verwendung in der Medizin. Es ist bevorzugt, die erfindungsgemäßen Verbindungen oder den Kit, der diese umfasst, in einer Kombinationstherapie, insbesondere zur Behandlung von Tumoren, zu verwenden. Besonders bevorzugt ist hierbei, dass die Kombinationstherapie eine Chemotherapie, eine Zytostatikabehandlung und/oder eine Strahlentherapie umfasst. In einer besonders bevorzugten Ausführungsform der Erfindung ist die Kombinationstherapie eine adjuvante biologisch-spezifizierte Therapieform. Ganz besonders bevorzugt ist hierbei, dass diese Therapieform eine Immuntherapie ist. Weiterhin ist besonders bevorzugt, dass die Kombinationstherapie eine Gentherapie und/oder eine Therapie mit einer erfindungsgemäßen Verbindung umfasst. Dem Fachmann sind verschiedene Kombinationstherapien, insbesondere zur Behandlung von Tumoren, bekannt. Es kann zum Beispiel vorgesehen sein, dass innerhalb einer Kombinationstherapie eine Zytostatikabehandlung erfolgt oder beispielsweise eine Bestrahlung eines bestimmten Tumorareals, wobei diese Behandlung mit einer Gentherapie kombiniert wird, wobei die erfindungsgemäßen Verbindungen als Antikrebsmittel eingesetzt werden. Demgemäß kann es ganz besonders bevorzugt sein, dass die erfindungsgemäßen Verbindungen zur Erhöhung der Sensitivität von Tumorzellen gegenüber Zytostatika und/oder Strahlen verwendet werden. Weiterhin ist es bevorzugt, dass die erfindungsgemäßen Verbindungen zur Hemmung der Vitalität, der Proliferationsrate von Zellen und/oder zur Induktion von Apoptose und eines Zellzyklus-Arrests verwendet werden.

Im Folgenden soll die Erfindung anhand von Beispielen näher erläutert werden, ohne auf diese Beispiele beschränkt zu sein.

### Beispiele

Allgemeine Darstellung der Gewinnung der erfindungsgemäßen Zusammensetzung mit dem erfindungsgemäßen Verfahren

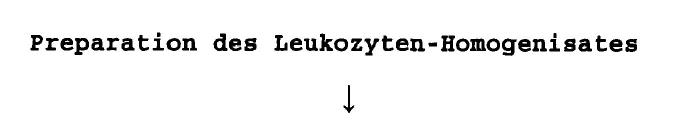

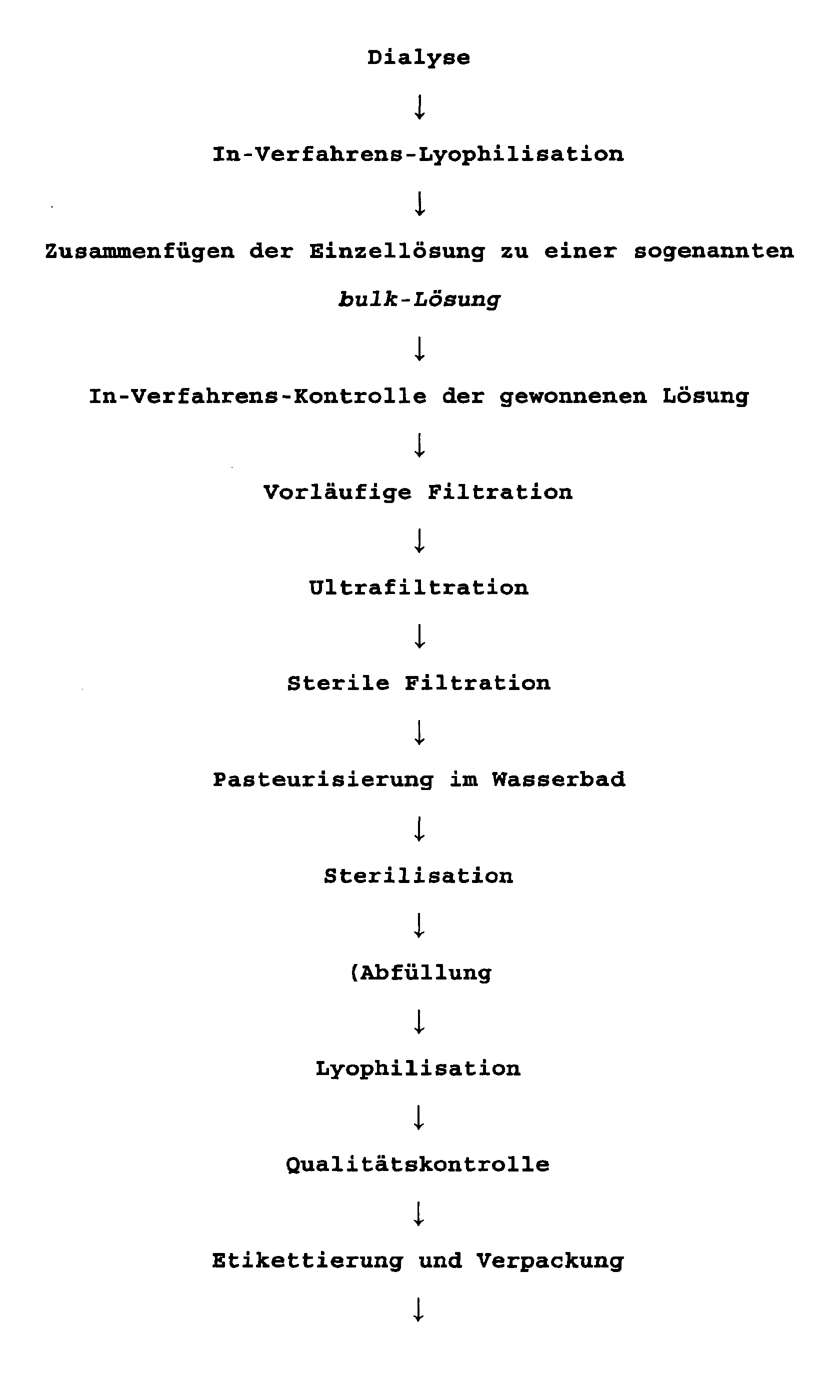

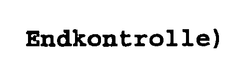

### Beschreibung des Verfahrens

1. Preparation eines Konzentrates, welches zelluläre Blutbestandteile, z.B. Leukozyten oder Erytrozyten, umfasst:
   Zunächst wird ein Homogenat aus ausgewählten Zellen präpariert, beispielsweise durch einen wiederholten Gefrier-Tau-Zyklus bzw. mittels Ultraschall-Behandlung der Zellen bzw. durch Kombination der beiden Prozesse. Anschließend werden die einzelnen Volumina gepoolt.
2. Dialyse des Homogenats:
   Die Dialyse wird als Säulen- bzw. Membran-Dialyse durchgeführt, bei der alle Partikel, die größer sind als 10 kDa, abgetrennt werden.
3. Zwischen-Lyophilisation:
   Das Dialysat wird durch Lyophilisation konzentriert, wobei die Lyophilisation mit üblichen Standardverfahren durchgeführt wird.
4. Preparation einer vorläufigen Lösung der erfindungsgemäßen Zusammensetzung:
   Das Lyophilisat wird mit 2ml Aqua aufgefüllt.
5. Zwischenkontrolle:
   Die Zwischenkontrolle wird durch Absorptionsmessung im Spektrum von 260 bis 280nm durchgeführt.
6. Vorfiltration:
   Die Filtration erfolgt durch eine Millipore-Membran RA (1,2µm) mit einem Vorfilter AP 15.
7. Ultrafiltration:
   Die Ultrafiltration erfolgt durch eine PTGC Membran in einem Millipore-Kassetten-System mit einem Ausschluss-Limit von 10 KDa.
8. Sterilisation durch Filtration:
   Die Sterilisation durch Filtration erfolgt mittels eines Millipore-GS-Filters von (0,22µm).
9. Hitzeinaktivierung:
   Die erfindungsgemäße Lösung wird in einzelnen Gefäßen in einem Wasserbad bei einer Temperatur von 60°C über 10 Stunden pasteurisiert.
10. Aliquotierung:
   Die flüssige Zusammensetzung wird automatisch aliquotiert unter sterilen Bedingungen (von 2 Litern Gesamtlösung in 5ml-Fläschchen).
11. Lyophilisation:
   Die Lyophilisation erfolgt mit Standardverfahren. Das Abfüllen der einzelnen Aliquots erfolgt unter Stickstoffatmosphäre und Kühlung.
   Die erfindungsgemäße Zusammensetzung wurde auch in-vivo in verschiedenen Tiersystemen geprüft. Mit Hilfe des Rosettentests bei Meerschweinchen-T-Lymphozyten wurde der Status der zellulären Immunität unter Einfluß der erfindungsgemäßen Zusammensetzung in Kombination mit Oxoplatin, Campto, Taxol und Eloxatin untersucht. Es wurde jeweils die Verbesserung des T-Lymphozytenstatus in immunsupprimierten Meerschweinchen nach Gabe der erfindungsgemäßen Zusammensetzung getestet. Vor den Versuchen wurde die Menge der T-Lymphozyten durch den Rosetten-Test in den Meerschweinchen bestimmt und anschließend wurde die Abnahme der Menge der T-Lymphozyten durch das immunsupprimierende Mittel Azathioprin bestimmt. Eine zweite Bestimmung der T-Lymphozyten erfolgte sieben Tage nach der Applikation von Azathioprin. Hierauf folgte die subkutane Applikation der erfindungsgemäßen Zusammensetzung in die Labortiere. Die letzte Bestimmung der Anzahl der T-Lymphozyten in den Meerschweinchen erfolgte 14 bis 19 Tage nach der Applikation der erfindungsgemäßen Zusammensetzung.

Bei diesen in-vivo-Versuchen zeigte sich, dass die Produktion von Rosetten nach der Applikation von Oxoplatin um ca. 30% fiel. Die durchschnittliche Erhöhung der Bildung der Rosetten nach der Applikation mit der erfindungsgemäßen Zusammensetzung betrug 27% bei einfacher Oxoplatin-Gabe und 23%, wenn die Tiere mit der doppelten Menge Oxoplatin vorab behandelt wurden.

Bei den Versuchen, bei denen Campto und die Zusammensetzung verwendet wurden, zeigte sich, dass die Produktion der Rosetten nach Applikation von Campto um 23% vermindert wurde und nach der Applikation der Zusammensetzung um 26% stieg.

Sofern Taxol und die Zusammensetzung eingesetzt wurden, zeigte sich ein Abfall der Produktion der Rosetten um 16% und eine Steigerung nach Gabe der erfindungsgemäßen Zusammensetzung um 23%.

Die Formation der Rosetten nach Gabe von Eloxatin sank um 22% und stieg nach Gabe der erfindungsgemäßen Zusammensetzung um 29%.

Zur weiteren Verifizierung der Effekte der erfindungsgemäßen Zusammensetzung wurden auch humane klinische Studien durchgeführt:

Es wurden fünf weibliche Patienten, die eine Sklerosis (PSS) aufwiesen, mit der Zusammensetzung behandelt. Eine Normalisierung der Zahl der Rosettenzellen konnte festgestellt werden. Neben der Normalisierung der zellulären Immunität konnte auch eine Verbesserung der acralen Zirkulation nachgewiesen werden. Der klinische Effekt der Zusammensetzung bei der Normalisierung des Levels der Rosettenzellen führte zu einer Steigerung von 38% zu 64%.

Weiterhin wurde die erfindungsgemäße Zusammensetzung bei humanen Patienten untersucht, die Psoriasis vulgaria und eine Arthritis, die auf Psoriasis zurückgeht, untersucht. Patienten wurden drei Dosen der erfindungsgemäßen Zusammensetzung in wöchentlichen Intervallen gegeben, wobei eine Dosis 4 mg Zusammensetzung in 2 ml umfasste. Nach sechs Monaten nach Beginn der Therapie zeigte sich bei drei von acht Patienten das vollständige Verschwinden der Syndrome der beiden genannten Krankheiten. In fünf weiteren Fällen wurde eine signifikante Verbesserung des Krankheitsbildes beobachtet. Die immunologische Verbesserung der Gesamtsituation war verbunden mit einer Verbesserung der Gesamtheit der relevanten klinischen Daten. Das Durchnittslevel der Rosettenzellen betrug bei den Patienten vor dem Start der Therapie 33% und stieg auf 67% zum Ende der Therapie.
In einem weiteren klinischen Versuch wurde die erfindungsgemäße Zusammensetzung bei weiblichen Patienten eingesetzt, bei denen Systemischer Lupus Erythematosus (SLE) diagnostiziert wurde. Bei den Patienten konnte eine Verbesserung des Krankheitsbildes festgestellt werden. Einige Patienten wiesen zusätzlich eine Infektion mit Candida-Erregern auf. Auch bei diesen Patienten wurde eine Verbesserung der klinischen Gesamtsituation dergestalt festgestellt, dass der Beginn einer Standardtherapie möglich war.

Weiterhin wurden Untersuchungen mit 35 Patienten durchgeführt, bei denen eine anteriore Unveitis diagnostiziert wurde. Die Dauer der Behandlung betrug 35 bis 85 Monate, wobei der Erfolg der Therapie nochmals im Zeitraum von 145 bis 195 Monaten untersucht wurde. Es konnte gezeigt werden, dass diese Langzeit-Applikation bei 90% der Patienten ein erneutes Auftreten der anterioren Unveitis verhinderte. Bei den Patienten, bei denen ein erneutes Auftreten der Krankheit diagnostiziert wurde, trat diese Krankheit dann in sehr milder Form auf. Ca. 5% der Patienten zeigten keine Reaktion auf die Behandlung.

Es wurden mit der erfindungsgemäßen Zusammensetzung weiterhin verschiedene Krebszelllinien getestet, wobei einzelne Verdünnungsschritte, beginnend mit 500 µg/ml untersucht wurden. Die besten Effekte wurden bei einer Konzentration von 30 µg/ml bestimmt.

| **Zelllinie** | | **% Inhibition** |
|---|---|---|
| Colo205 | Dickdarm | 10,9 |
| BT20 | Brust | 0 |
| PC3 | Prostata | - 7,4 |
| SK-MTC | Schilddrüse | 16,6 |
| J82 | Blase | 8,7 |
| WI38 | Fibroblasten | 14,5 |
| A431 | Karzinome | 13,3 |
| HT29 | Dickdarm | 9,4 |
| PANC1 | Pankreas | 17,0 |
| MIAPaCa2 | Pankreas | 34,9 |
| LNCaP | Prostata | 30,8 |
| NIH3T3 | Fibroblasten | 4,8 |

Die erfindungsgemäße Zusammensetzung hat einen antiproliferativen Effekt auf die meisten Zelllinien, insbesondere bei MIAPaCa-2 Pankreaskrebszellen sowie bei LNCaP Hormon-sensitiven Prostatakrebszelllinien.

## Patentansprüche

1. Verfahren zur Herstellung einer Zusammensetzung zur Behandlung von Defekten der zellulären Immunität in einem Patienten, umfassend die Schritte einer Homogenisation von zellulären Blutbestandteilen, insbesondere Leukozyten, einer Lyophilisation des Homogenats und einer Abtrennung von Bestandteilen mit einem Molekulargewicht von über 10.000 Da.

2. Verfahren nach Anspruch 1, **dadurch gekennzeichnet, dass** zunächst ein Leukozytenkonzentrat hergestellt wird, welches anschließend dialysiert, folgend vorfiltriert, ultrafiltriert und bevorzugt anschließend pasteurisiert wird.

3. Verfahren nach Anspruch 1 oder 2, **gekennzeichnet durch**
a) Homogenisierung zellulärer Blutbestandteile **durch** einen Gefrier-Tau-Zyklus und/oder eine Ultraschallbehandlung;
b) Lyphilisation eines Homogenats erhalten gemäß a;
c) Vorfiltration eines Lyophilisats erhalten gemäß b;
d) Ultrafiltration eines Vorfiltrates erhalten gemäß c;
e) Sterilfiltration eines Ultrafiltrates erhalten gemäß d;
f) Pasteurisierung eines sterilen Filtrates erhalten gemäß e;
g) Sterilisierung eines pasteurisierten Produktes gemäß f) und
h) Lyophilisation eines sterilen Produktes gemäß g).

4. Verfahren nach einem der vorhergehenden Ansprüche, **dadurch gekennzeichnet, dass** die Lyophilisation unter Zusatz von Lösungen erfolgt, insbesondere unter Zusatz von Puffern, Salzen, Vitaminen, Zuckerderivaten, Enzymen und/oder Pflanzenextrakten.

5. Verfahren nach einem der vorhergehenden Ansprüche, **dadurch gekennzeichnet, dass** weiterhin die Formulierung der gewonnenen Zusammensetzung oder eines Derivates oder eines Homologen hiervon mit einem pharmazeutisch akzeptablen Träger umfasst.

6. Zusammensetzung, gewinnbar mit einem Verfahren gemäß der Ansprüche 1 bis 5.

7. Zusammensetzung nach Anspruch 6, umfassend Ubiquitin spezifische Protease 32, positiver Kofaktor 2 Glutamin/Q-reiches assoziiertes Protein, Cadherin, Transskriptionsfakor GATA-2, putativer Chromatin-Struktur-Regulator, CUE Domain - enthaltend 1, SUPT6H-Protein, Interleukin 18 Rezeptor 1 Vorläufer, Interleukin 1 Rezeptor-ähnliches Protein und Mucin 4 und/oder Tenascin M, transienter Rezeptor potentiellen Kation-Kanals, Ectonucleotid-Pyrophosphatase/Phospodiesterase, SWI-/SNF-verwandtes matrixassoziiertes Actin-abhängiger Regulator des Chromatins, SWI/SNF Chromatin-modumulierende Komplex-Untereinheit OSA1 B120, OSA1 Kernprotein, ATPase, H+/K+ Wechselpolypeptid, Zink-Finger-Protein 174, Ig-schwere Ketten-V-Region, ADAMTS-3 Vorläufer/Desintegrin-ähnliche und Metalloprotease, Koagulationsfaktor II (Thrombin)Rezeptor-ähnlich 3, Diacylglycerol Kinase-theta, p250R, SWI/SNF Chromatin-remodulierende Komplex-Untereinheit OSA2, Metallo-phospoesterase, AMP-Deaminase, Alpha-Mannosidase, Cadherin 9, Nik-verwandte Kinase, Alhpa-1B-adrenerger Rezeptor, BRCA1-assoziiertes Protein-1, CD99 Antigen-ähnliche 2 isoform E3-E4, SWHCF-umfassendes Peptid und/oder FAT-ähnliches Cadherin-FATJ, ATP-Bindungskassette, Nucleoporin 153 kDa, ELK3-Protein, Protein ELK-3 ETS-Domäne, ATPase, Kupfer-transportierende Protein-Kinase, Protein Tyrosin-Phosphatase, Integrationsseiten-Familie flügelloser Typ MMTV, MYC Bindungsprotein 2, Cullin 7, gelöste Carrier-Familie 5 (Natriumjodid-Symporter) Mitglied 5, Glutamat-reiche WD Wiederholung enthaltend 1, MAP Kinase interagierende Serin/Threonin Kinase 1, ATP-Bindungskassette, NOV-Plexin-A1 Protein und/oder E3 Ubiquitin Ligase SMURF2, Acyl-CoA Synthetase, Estrogen Sulfotransferase, 2,4-Dienoyl CoA Reductase 1 Vorläufer, 4-Enoyl-CoA Reductase, Claudin 10 Isoform b, DMBT1, extrazellulärer Linker-Domäne enthaltend 1, lymphatischer Endothel-spezifischer Hyaluron-Rezeptor LYVE-1, Rho GTPase aktivierendes Protein 8 Isoform 2, Desintegrin-ähnliches und Metalloprotease (Reprolysintyp) mit Thrombospondin Typ 1 Motiv, Ig-schwere Ketten-V-Region, AS12-Protein, mitochondriales ribosomales Protein S9, 28S ribosomales Protein S9, Protein-Kinase-Substrat MK2S4, NP220, putativer G-Protein-gekoppelter Rezeptor, Dynein-, Axonemal-schweres Polypeptid 5, N-acyl-phosphatidylethanolamin-hydrolysierende Phospholipase D, Leukotrein-B4-Rezeptor, G-Protein-gekoppelter Rezeptor 16, Proprotein Convertase Subtilisin/Kexin Typ-1-Inhibitor CMKRL1, dualspezifische Tyrosin-phosphorylierungs-regulierte Kinase 3, regulatorische Erythroid-Kinase (Langform), DYRK3-Protein, Ig lambda Kette V-VII Region (Mot) - human, Glutathion-Reduktase, mitochondrialer Vorläufer, Collagen alpha 1 (XVI) Kettenvorläuer, 11-beta-Hydroxysteroid Dehydrogenase 1, Insulin-Rezeptor Substrat 2, Vault poly (ADP-ribose) Polymerase (VPARP), Calcium-/Calmodulin-abhängige 3', 5'-zyklische Nucleotide, Zinkfinger-Protein 161, H2.0-ähnliche Homeo-Box-1, H2.0 (Drosophilia)-ähnliche Homeo-Box-1 und/oder Dedicator der Zytokinese 6.

8. Zusammensetzung nach Anspruch 7, umfassend Interleukin 18 Rezeptor 1 Vorläufer, Interleukin 1 Rezeptor-ähnliches Protein und Mucin 4, transienter Rezeptor potentiellen Kation-Kanals, Ectonucleotid-Pyrophosphatase/Phospodiesterase, SWI-/SNF-verwandtes matrixassoziiertes Actin-abhängiger Regulator des Chromatins, SWI/SNF Chromatin-modumulierende Komplex-Untereinheit OSA1 B120, OSA1 Kernprotein, MYC Bindungsprotein 2, Cullin 7, gelöste Carrier-Familie 5 (Natriumjodid-Symporter) Mitglied 5, Glutamat-reiche WD Wiederholung enthaltend 1, MAP Kinase interagierende Serin/Threonin Kinase 1, ATP-Bindungskassette, DMBT1, extrazellulärer Linker-Domäne enthaltend 1, lymphatischer Endothel-spezifischer Hyaluron-Rezeptor LYVE-1, Rho GTPase aktivierendes Protein 8 Isoform 2, Desintegrin-ähnliches und Metalloprotease (Reprolysintyp) mit Thrombospondin Typ 1 Motiv, AS12-Protein, mitochondriales ribosomales Protein S9, 28S ribosomales Protein S9, Protein-Kinase-Substrat MK2S4, NP220, putativer G-Protein-gekoppelter Rezeptor, Dynein-, Axonemal-schweres Polypeptid 5, N-acyl-phosphatidylethanolamin-hydrolysierende Phospholipase D, Leukotrein-B4-Rezeptor, G-Protein-gekoppelter Rezeptor 16, Proprotein Convertase Subtilisin/Kexin Typ-1-Inhibitor CMKRL1, dualspezifische Tyrosin-phosphorylierungs-regulierte Kinase 3, regulatorische Erythroid-Kinase (Langform), DYRK3-Protein und/oder Ig lambda Kette V-VII Region (Mot) - human.

9. Pharmazeutisches Mittel umfassend die Zusammensetzung nach Anspruch 6 bis 8, gegebenenfalls zusammen mit einem pharmazeutisch akzeptablen Träger.

10. Pharmazeutisches Mittel nach dem vorhergehenden Anspruch, **dadurch gekennzeichnet, dass** der Träger ausgewählt ist aus der Gruppe umfassend Füllmittel, Sprengmittel, Bindemittel, Feuchthaltemittel, Streckmittel, Lösungsverzögerer, Resorptionsbeschleuniger, Netzmittel, Absorptionsmittel und/oder Gleitmittel.

11. Pharmazeutisches Mittel nach einem der vorhergehenden Ansprüche, **dadurch gekennzeichnet, dass** es eine Kapsel, eine Tablette, ein Dragee, ein Zäpfchen, eine Salbe, eine Creme, eine Injektionslösung und/oder eine Infusionslösung ist.

12. Pharmazeutisches Mittel nach einem der vorhergehenden Ansprüche, **dadurch gekennzeichnet, dass** es ein Vaginal-, Rektalzäpfchen, -schwämmchen und/oder -schaum ist.

13. Pharmazeutisches Mittel nach einem der vorhergehenden Ansprüche, **dadurch gekennzeichnet, dass** es in Liposomen, Siosomen und/oder Niosomen eingeschlossen vorliegt.

14. Kit umfassend eine Zusammensetzung nach Anspruch 6 bis 8 und/oder eine pharmazeutische Zusammensetzung nach einem der Ansprüche 9 bis 13, gegebenenfalls zusammen mit einer Information über die Kombination und/oder Handhabung der Bestandteile des Kits.

15. Verwendung der Zusammensetzung nach Anspruch 6 bis 8 und/oder des pharmazeutischen Mittels nach einem der Ansprüche 9 bis 13 zur Herstellung eines Arzneimittels zur Behandlung von pathogenen Modifikationen der zellulären Immunität in einem Patienten, insbesondere einem Defekt der zellulären Immunität, insbesondere nach ICD10-Code: D84.8.

16. Verwendung nach dem vorhergehenden Anspruch, **dadurch gekennzeichnet, dass** das Arzneimittel im Zusammenhang mit einer Zytostatika-, Chemo- und/oder Strahlungstherapie mit dem Patienten in Kontakt gebracht wird.

17. Verwendung nach dem vorhergehenden Anspruch, **dadurch gekennzeichnet, dass** das Inkontaktbringen oral, vaginal, rektal, nasal, topisch, subkutan, intravenös, intramuskulär, intraperitoneal über Injektionen und/oder über Infusionen erfolgt.

18. Verwendung nach einem der vorhergehenden Ansprüche, **dadurch gekennzeichnet, dass** das Arzneimittel vor und/oder nach schweren Unfällen mit Personen, Tieren und/oder einem Patienten in Kontakt gebracht wird, insbesondere zur Prophylaxe von Folgedefekten, bevorzugt Sepsis.

19. Verwendung nach einem der vorhergehenden Ansprüche zur Prophylaxe und Therapie im Zusammenhang mit einem Unfall mit atomaren, biologischen, chemischen und/oder radioaktiven Stoffen und/oder Materialien, insbesondere wenn Personen und/oder Tiere mit diesen in Kontakt gebracht worden sind.

## Geänderte Patentansprüche

### Geänderte Patentansprüche gemäss Regel 86(2) EPÜ.

**4.** Verfahren nach einem der vorhergehenden Ansprüche, **dadurch gekennzeichnet, dass** die Lyophilisation unter Zusatz von Lösungen erfolgt, insbesondere unter Zusatz von Puffern, Salzen, Vitaminen, Zuckerderivaten, Enzymen und/oder Pflanzenextrakten.

**5.** Verfahren nach einem der vorhergehenden Ansprüche, **dadurch gekennzeichnet, dass** weiterhin die Formulierung der gewonnenen Zusammensetzung oder eines Derivates oder eines Homologen hiervon mit einem pharmazeutisch akzeptablen Träger umfasst.

**6.** Zusammensetzung, gewinnbar mit einem Verfahren gemäß der Ansprüche 1 bis 5, wobei Leukozyten homogenisiert werden.

**7.** Zusammensetzung nach Anspruch 6, umfassend Ubiquitin spezifische Protease 32, positiver Kofaktor 2 Glutamin/Q-reiches assoziiertes Protein, Cadherin, Transskriptionsfakor GATA-2, putativer Chromatin-Struktur-Regulator, CUE Domain - enthaltend 1, SUPT6H-Protein, Interleukin 18 Rezeptor 1 Vorläufer, Interleukin 1 Rezeptor-ähnliches Protein und Mucin 4 und/oder Tenascin M, transienter Rezeptor potentiellen Kation-Kanals, Ectonucleotid-Pyrophosphatase/Phospodiesterase, SWI-/SNF-verwandtes matrixassoziiertes Actin-abhängiger Regulator des Chromatins, SWI/SNF Chromatin-modumulierende Komplex-Untereinheit OSA1 B120, OSA1 Kernprotein, ATPase, H+/K+ Wechselpolypeptid, Zink-Finger-Protein 174, Ig-schwere Ketten-V-Region, ADAMTS-3 Vorläufer/Desintegrinähnliche und Metalloprotease, Koagulationsfaktor II (Thrombin)Rezeptor-ähnlich 3, Diacylglycerol Kinasetheta, p250R, SWI/SNF Chromatin-remodulierende Komplex-Untereinheit OSA2, Metallo-phospoesterase, AMP-
